(19) Europäisches Patentamt
European Patent Office
Office européen des brevets

(11) Veröffentlichungsnummer: **0 079 556**
A2

(12) **EUROPÄISCHE PATENTANMELDUNG**

(21) Anmeldenummer: **82110285.2**

(22) Anmeldetag: **08.11.82**

(51) Int. Cl.³: **C 07 D 221/28**
A 61 K 31/435, C 07 D 405/04
C 07 D 405/06
//(C07D405/04, 319/00, 221/00),
(C07D405/04, 317/00, 221/00),
(C07D405/06, 307/00, 221/00)

(30) Priorität: **09.11.81 US 319482**

(43) Veröffentlichungstag der Anmeldung:
**25.05.83 Patentblatt 83/21**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Mohacsi, Ernest**
**Franklin Avenue 640**
**Nutley, N.J. 07110(US)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) **Morphinanderivate, ihre Herstellung, Zwischenprodukte für ihre Herstellung, sowie diese enthaltende Arzeimittel.**

(57) Es werden neue pharmazeutische Wirkstoffe mit wertvollen analgetischen Eigenschaften beschrieben, welche in Form von pharmazeutischen Präparaten als Heilmittel verwendet werden können. Es handelt sich dabei um Morphinanderivate der Formel

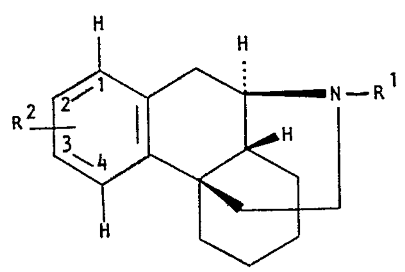

I

worin $R^1$ Wasserstoff, niederes Alkyl, niederes Alkenyl, niederes Cycloalkyl-niederes Alkyl, Cyano-niederes Alkyl, Aryl-niederes Alkyl oder Heteroaryl-niederes Alkyl, $R^2$ $(C_{2-7})$-Alkanoyl, Arylcarbonyl, Trifluormethylcarbonyl oder die Gruppe RR'C(OH)−, R $(C_{1-6})$-Alkyl und R' Wasserstoff oder niederes Alkyl bedeuten,
und ihre pharmazeutisch annehmbaren Säureadditionssalze.

EP 0 079 556 A2

F.Hoffmann-La Roche & Co.Aktiengesellschaft, Basel/Schweiz



RAN 4001/106

# Morphinanderivate, ihre Herstellung, Zwischenprodukte für ihre Herstellung, sowie diese enthaltende Arzneimittel.

Die vorliegende Erfindung betrifft Morphinanderivate der allgemeinen Formel

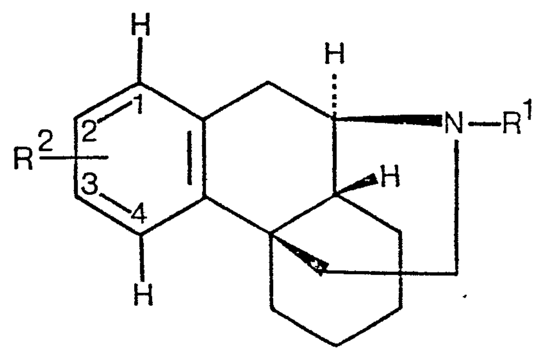

I

worin $R^1$ Wasserstoff, niederes Alkyl, niederes Alkenyl, niederes Cycloalkyl-niederes Alkyl, Cyano-niederes Alkyl, Aryl-niederes Alkyl oder Hetero-aryl-niederes Alkyl, $R^2$ $(C_{2-7})$-Alkanoyl, Arylcarbonyl, Trifluormethylcarbonyl oder die Gruppe RR'C(OH)-, R $(C_{1-6})$-Alkyl und R' Wasserstoff oder niederes Alkyl bedeuten,

und pharmazeutisch annehmbare Säureadditionssalze davon. Diese Verbindungen können als Analgetika verwendet werden.

Der Ausdruck "niederes Alkyl" bezeichnet geradkettige und verzweigte, gesättigte Kohlenwasserstoffreste mit 1-7 Kohlenstoffatomen, wie Methyl, Aethyl, n-Propyl,

Nt/5.10.82

Isopropyl, n-Butyl, Isobutyl und dergleichen, wobei Methyl bevorzugt ist. Der Ausdruck "niederes Alkenyl" bezeichnet geradkettige und verzweigte Kohlenwasserstoffreste mit 2-7 Kohlenstoffatomen, welche eine olefinische Doppelbindung enthalten, wie Vinyl, Allyl, 1-Propenyl, 3-Methyl-2-butenyl, 2-Methyl-2-propenyl und dergleichen. Bevorzugte niedere Alkenylgruppen sind Allyl, 3-Methyl-2-butenyl und 2-Methyl-2-propenyl.

Der Ausdruck "niederes Cycloalkyl" bezeichnet gesättigte cyclische Kohlenwasserstoffreste mit 3-6 Ringgliedern. Unter den bevorzugten niederen Cycloalkylgruppen sind Cyclopropyl, Cyclobutyl und Cyclohexyl. Der Ausdruck "($C_{2-7}$)-Alkanoyl" bezeichnet einen von einer aliphatischen Carbonsäure mit 2-7 Kohlenstoffatomen abgeleiteten Rest. Unter den bevorzugten ($C_{2-7}$)-Alkanoylgruppen sind Acetyl und dergleichen. Der Ausdruck "aromatisch" oder "Aryl" bezeichnet Kohlenwasserstoffringsysteme, wie Phenyl und Naphthyl, wobei Phenyl bevorzugt ist. Der Ausdruck "Arylcarbonyl" bezeichnet einen von einer aromatischen Carbonsäure abgeleiteten Rest. Unter den bevorzugten Arylcarbonylgruppen sind Benzoyl, Naphthoyl und dergleichen.

Der Ausdruck "Heteroaryl" bezeichnet 5- oder 6-gliedrige aromatische Heterozyklen, welche ein Sauerstoff-, Stickstoff- oder Schwefelatom als Heteroatom enthalten, vorzugsweise Thienyl, Pyrrolyl, Furyl, Pyridyl, Pyranyl und dergleichen.

Eine bevorzugte Untergruppe von Verbindungen der Formel I sind solche, worin $R^1$ niederes Alkyl, niederes Alkenyl, niederes Cycloalkyl-niederes Alkyl oder Aryl-niederes Alkyl bedeutet und $R^2$ sich in Stellung 3 befindet und ($C_{2-7}$)-Alkanoyl bedeutet.

Eine weitere bevorzugte Untergruppe umfasst Verbindungen der Formel I, worin $R^1$ niederes Alkyl bedeutet

und $R^2$ sich in Stellung 3 befindet und die Gruppe RR'C(OH)- bedeutet.

Bevorzugte erfindungsgemässe Verbindungen sind (-)-3-Acetyl-N-(cyclopropylmethyl)morphinan und (-)-3-(1-Hydroxyäthyl)-N-methylmorphinan.

Die Verbindungen der Formel I und ihre pharmazeutisch annehmbaren Säureadditionssalze können erfindungsgemäss dadurch hergestellt werden, dass man

a) eine Verbindung der allgemeinen Formel

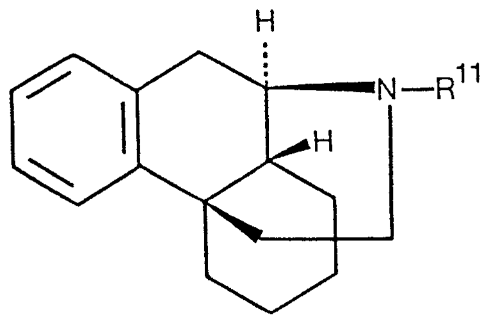

II

worin $R^{11}$ niederes Alkyl bedeutet,
acyliert, oder

b) eine Verbindung der allgemeinen Formel

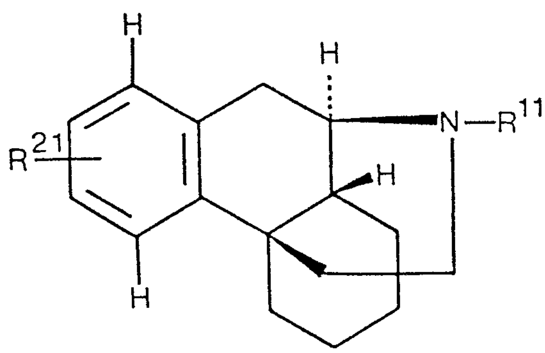

Ia

worin $R^{21}$ $(C_{2-7})$-Alkanoyl, Arylcarbonyl oder Trifluormethylcarbonyl bedeutet und $R^{11}$ obige Bedeutung besitzt,
N-dealkyliert, oder

c) eine Verbindung der allgemeinen Formel

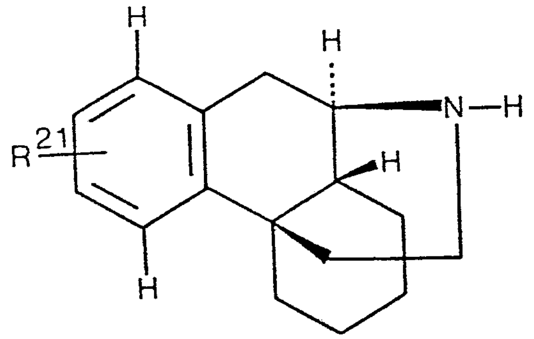

Ib

worin $R^{21}$ obige Bedeutung besitzt, mit einem eine niedere Alkyl-, niedere Alkenyl-, niedere Cycloalkyl-niedere Alkyl-, Cyano-niedere Alkyl-, Arylniedere Alkyl- oder Heteroaryl-niedere Alkylgruppe liefernden Alkylierungsmittel alkyliert, oder

d) die cyclische Ketalschutzgruppe in einer Verbindung der allgemeinen Formel

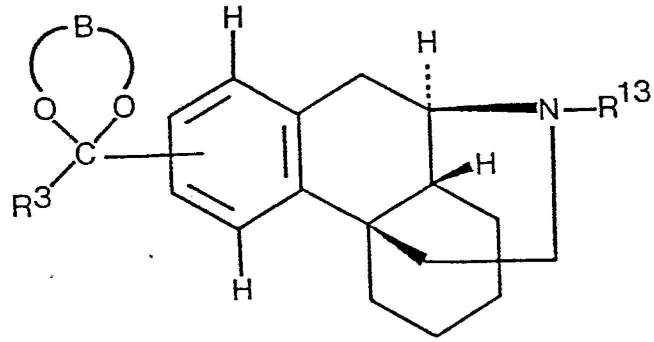

III

worin $R^{13}$ niederes Alkyl, niederes Cycloalkyl-niederes Alkyl, Aryl-niederes Alkyl oder Heteroaryl-niederes Alkyl, B Dimethylen oder Trimethylen und $R^3$ $(C_{1-6})$-Alkyl, Aryl oder Trifluormethyl bedeuten,

abspaltet, oder

e) die Carbonylgruppe in einer Verbindung der allgemeinen Formel

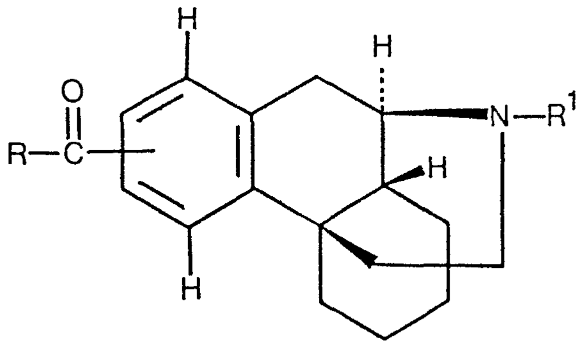

Ic

worin R und $R^1$ obige Bedeutung besitzen, reduziert, oder

f) die Carbonylgruppe in einer Verbindung der obigen Formel Ic alkyliert und

g) erwünschtenfalls eine erhaltene Verbindung der Formel I in ein pharmazeutisch annehmbares Säureadditionssalz überführt.

Gemäss Verfahrensvariante a) kann man eine Verbindung der Formel II am aromatischen Ring acylieren, und zwar vorzugsweise durch Umsetzen mit einem Acylierungsmittel, wie einem Säurehalogenid, einem Säureanhydrid oder einer Carbonsäure und unter den Bedingungen einer Friedel-Crafts-Reaktion. Beispiele solcher Acylierungsmittel sind Verbindungen der allgemeinen Formel

$$R^3-\overset{O}{\overset{\|}{C}}-O-\overset{O}{\overset{\|}{C}}-R^3 \quad \text{und} \quad R^3-\overset{O}{\overset{\|}{C}}-Y,$$

worin $R^3$ $(C_{1-6})$-Alkyl, Aryl oder Trifluormethyl und Y Halogen oder Hydroxy bedeuten. Die Acylierung kann mit oder ohne Lösungsmittel durchgeführt werden. Als Lösungsmittel können z.B. 1,2-Dichloräthan, Nitrobenzol, Nitromethan, Methylenchlorid, Schwefelkohlenstoff und dergleichen verwendet werden. In der Friedel-Crafts-Reaktion können als Lewis-Säuren z.B. Aluminiumtrichlorid, Antimonpentachlorid, Eisentrichlorid, Bortrifluorid, Zinntrichlorid, Antimontrichlorid, Aluminiumtribromid, Bortrichlorid, Titantetrachlorid, Zinkchlorid und dergleichen

verwendet werden.

Die Acylierung eines aromatischen Systems nach Friedel-Crafts wird üblicherweise entweder mit einem Säurechlorid oder einem Anhydrid in Gegenwart einer Lewis-Säure, wie Aluminiumtrichlorid, Bortrifluorid, Zinntrichlorid, Zinkchlorid, Eisentrichlorid oder dergleichen, oder mit einer Carbonsäure in Gegenwart einer Säure, wie Fluorwasserstoff, Schwefelsäure, Phosphorsäure oder Polyphosphorsäure, durchgeführt. Die Lewis-Säure-Katalysatoren verwendet man üblicherweise in Lösungsmitteln, wie 1,2-Dichloräthan, Schwefelkohlenstoff, Methylenchlorid, Nitromethan, Nitrobenzol oder in einem Ueberschuss an zu acylierendem Kohlenwasserstoff. In der Regel führt man die Acylierung in einem Temperaturbereich von 0°C bis zum Siedepunkt des Lösungsmittels durch.

Die Reaktionsprodukte werden durch die Formeln I'a und I"a, charakterisiert, vgl. Reaktionsschema I, und können nach an sich bekannten Methoden, wie Chromatographie, Kristallisation und dergleichen, getrennt werden.

Die als Ausgangsstoffe verwendeten Verbindungen der Formel II sind bekannt oder können nach an sich bekannten Methoden hergestellt werden.

Reaktionsschema I

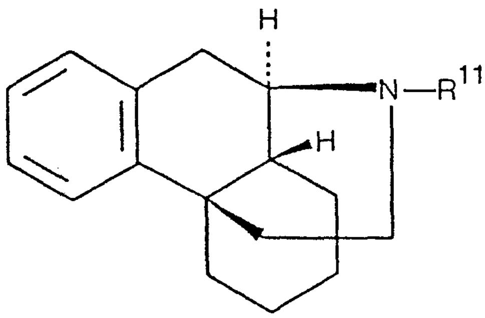

II

Acylierung

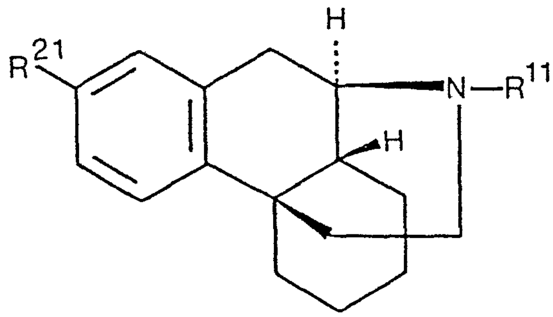

1'a

+

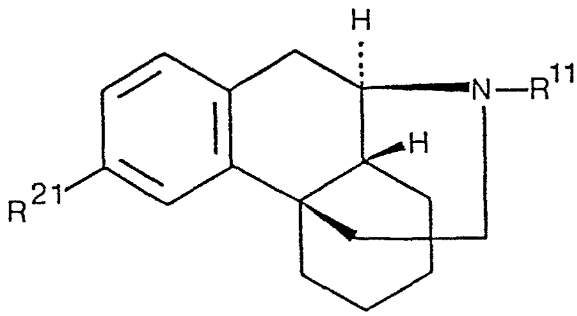

1"a

worin $R^{11}$ und $R^{21}$ obige Bedeutung besitzen.

Gemäss Verfahrensvariante b) kann man eine Verbindung der Formel Ic dealkylieren. Diese Umwandlung kann nach an sich bekannten Methoden erfolgen. Man kann dabei jedes herkömmliche Dealkylierungsmittel verwenden. Man kann eine Verbindung der Formel Ic beispielsweise zuerst mit Bromcyan und dann mit einer anorganischen Säure oder zuerst mit Phenyl- oder Aethylchloroformat und dann mit einem Alkalimetallhydroxid in einem niederen Alkohol behandeln.

In einer bevorzugten Ausführungsform, vgl. Reaktionsschema II, überführt man eine Verbindung der Formel Ia durch Behandeln mit Trichloräthylchloroformat in einem inerten Lösungsmittel, wie Benzol, Toluol oder dergleichen, vorzugsweise bei der Rückflusstemperatur der Reaktionsmischung in eine Verbindung der Formel IV. Durch Behandeln einer Verbindung der Formel IV mit Zink in einer niederen Alkancarbonsäure, wie Essigsäure, Propionsäure oder dergleichen, bei Raumtemperatur und Atmosphärendruck erhält man eine Verbindung der Formel Ib.

Gemäss Verfahrensvariante c), vgl. ebenfalls Reaktionsschema II, kann man eine Verbindung der Formel Ib durch Behandeln mit einem Alkylierungsmittel in eine Verbindung der Formel Id überführen. Beispiele von Alkylierungsmitteln sind Verbindungen der Formel $R^{12}$-X, worin X Halogen und $R^{12}$ niederes Alkyl, niederes Alkenyl, niederes Cycloalkyl-niederes Alkyl, Cyano-niederes Alkyl, Aryl-niederes Alkyl oder Heteroaryl-niederes Alkyl bedeuten. Für die Alkylierung bevorzugte Lösungsmittel sind Dimethylsulfoxid, Dimethylformamid und dergleichen. Die Reaktion kann in Gegenwart einer anorganischen Alkalimetallbase, wie Natrium- oder Kaliumcarbonat oder -bicarbonat und in einem Temperaturbereich von Raumtemperatur bis Rückflusstemperatur, vorzugsweise bei der Rückflusstemperatur, durchgeführt werden.

Reaktionsschema II

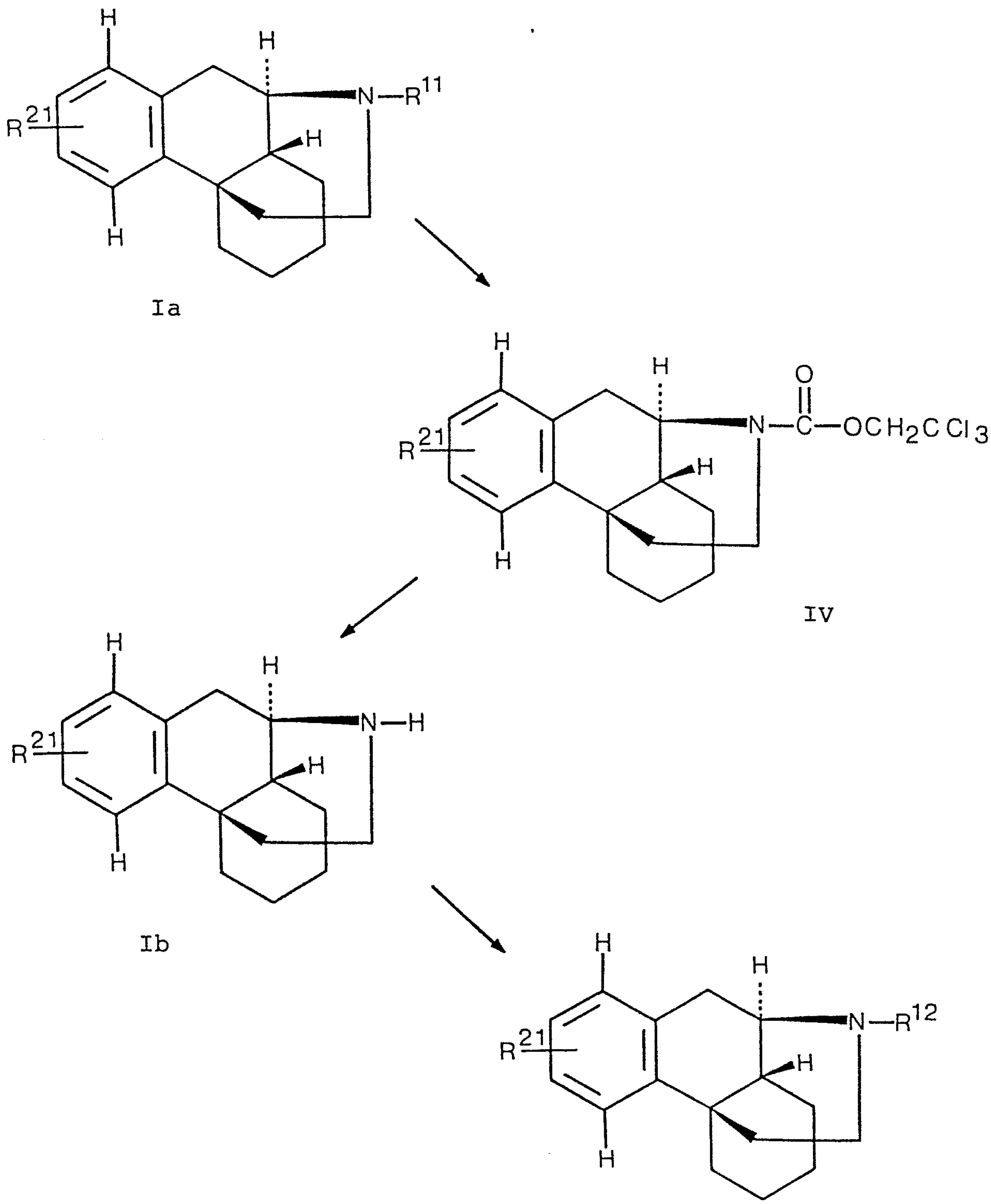

Ia

IV

Ib

Id

worin $R^{11}$, $R^{12}$ und $R^{21}$ obige Bedeutung besitzen.

Gemäss Verfahrensvariante d) kann man die cyclische Ketalschutzgruppe, vorzugsweise eine 1,2-Aethylenketalgruppe, in einer Verbindung der Formel II abspalten, wobei man eine Verbindung der Formel I erhält. Diese Reaktion kann durch Behandeln einer Verbindung der Formel III mit einer Mineralsäure, wie Salzsäure, Bromwasserstoff, Schwefelsäure oder dergleichen, durchgeführt werden.

Die als Ausgangsstoffe verwendeten Verbindungen der Formel III können ausgehend von Verbindungen der Formel Ib gemäss nachfolgendem Reaktionsschema III hergestellt werden:

Reaktionsschema III

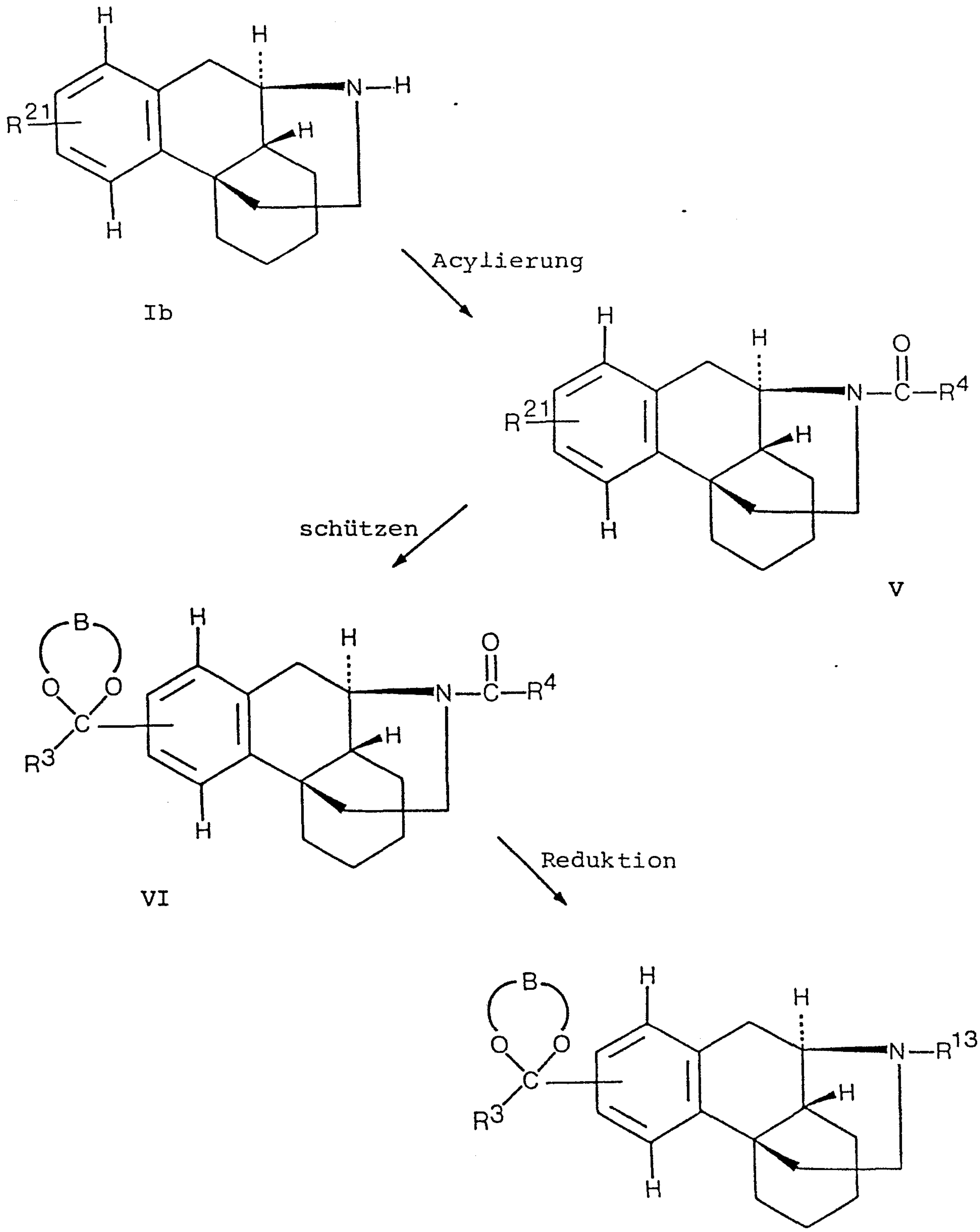


Ib

V

VI

III

worin $R^{13}$, $R^{21}$, $R^{3}$ und B obige Bedeutung besitzen und $R^{4}$ Wasserstoff, $(C_{1-6})$-Alkyl, niederes Cycloalkyl, niederes Cycloalkyl-$(C_{1-6})$-Alkyl, Aryl, Aryl-$(C_{1-6})$-Alkyl, Heteroaryl oder Heteroaryl-$(C_{1-6})$-Alkyl bedeutet.

Man kann eine Verbindung der Formel Ib mit einem Acylierungsmittel der Formel X-CO-$R^4$, worin X Halogen bedeutet und $R^4$ obige Bedeutung besitzt, in Gegenwart eines inerten organischen Lösungsmittels, wie Benzol, Toluol und Methylenchlorid, und in Gegenwart einer organischen Base, wie Pyridin, Triäthylamin oder dergleichen, bei Raumtemperatur oder bei der Siedetemperatur des Lösungsmittels acylieren. Als Acylierungsmittel kann man beispielsweise Cyclopropylcarbonylchlorid, Cyclobutylcarbonylchlorid, Phenylacetylchlorid, Furylacetylchlorid oder dergleichen verwenden.

Anschliessend kann man die im Rest $R^{21}$ vorhandene Carbonylgruppe in einer Verbindung der Formel V in Form eines cyclischen Ketals schützen. In der Regel stellt man Ketale aus Ketonen dadurch her, dass man die Carbonylverbindung in Gegenwart eines sauren Katalysators und unter azeotroper Entfernung des gebildeten Reaktionswassers mit einem mit Wasser nicht mischbaren Schlepper mit einem Alkandiol umsetzt. Man kann dabei jedes Alkandiol, wie Aethylenglykol, Propylenglykol und dergleichen, verwenden. Als Lösungsmittel kann man beispielsweise Benzol, Toluol, Xylol und dergleichen verwenden. Als Katalysator kann man p-Toluolsulfonsäure oder dergleichen verwenden.

Die Reduktion der Amidgruppe in einer Verbindung der Formel VI zum entsprechenden Amin kann mit einem Alkalimetallaluminiumhydrid, wie Lithiumaluminiumhydrid oder dergleichen, bewerkstelligt werden, wobei man bekannte Reaktionsbedingungen anwendet. Man kann beispielsweise in Gegenwart eines Lösungsmittels, wie Aether, Tetrahydrofuran, 1,2-Dimethoxyäthan, Diglym oder dergleichen, bei Raumtemperatur oder bei der Rückflusstemperatur arbeiten.

Gemäss Verfahrensvariante e), vgl. das nachfolgende Reaktionsschema IV, kann eine Verbindung der Formel Ic

dadurch in eine Verbindung der Formel Ie übergeführt werden, dass man ein Reduktionsmittel, wie Lithiumaluminiumhydrid, in Gegenwart eines Lösungsmittels, wie Aether, Tetrahydrofuran oder dergleichen, oder Natriumborhydrid in Gegenwart eines Lösungsmittels, wie Methanol, Aethanol oder dergleichen, verwendet und bei Raumtemperatur oder bei der Rückflusstemperatur des Lösungsmittels arbeitet.

Gemäss Verfahrensvariante f), vgl. ebenfalls Reaktionsschema IV, kann man eine Verbindung der Formel Ic mit einem Grignard-Reagens oder einer Organolithium-Verbindung alkylieren, wobei man eine Verbindung der Formel If erhält. Insbesondere kann man eine Verbindung der Formel Ic in einem Lösungsmittel, wie Diäthyläther oder Dibutyl- oder Diisopentyläther, bei Raumtemperatur oder bei der Rückflusstemperatur des Lösungsmittels mit einem Grignard-Reagens, wie einem Alkylmagnesiumhalogenid, umsetzen. Andererseits kann man eine Verbindung der Formel Ic auch mit einer Organolithium-Verbindung umsetzen. Beispielsweise kann man eine Verbindung der Formel Ic mit Methyllithium umsetzen, wobei man die weiter oben für die Grignard-Reaktion angegebenen Bedingungen verwendet und unter einer Stickstoffatmosphäre arbeitet.

Reaktionsschema IV

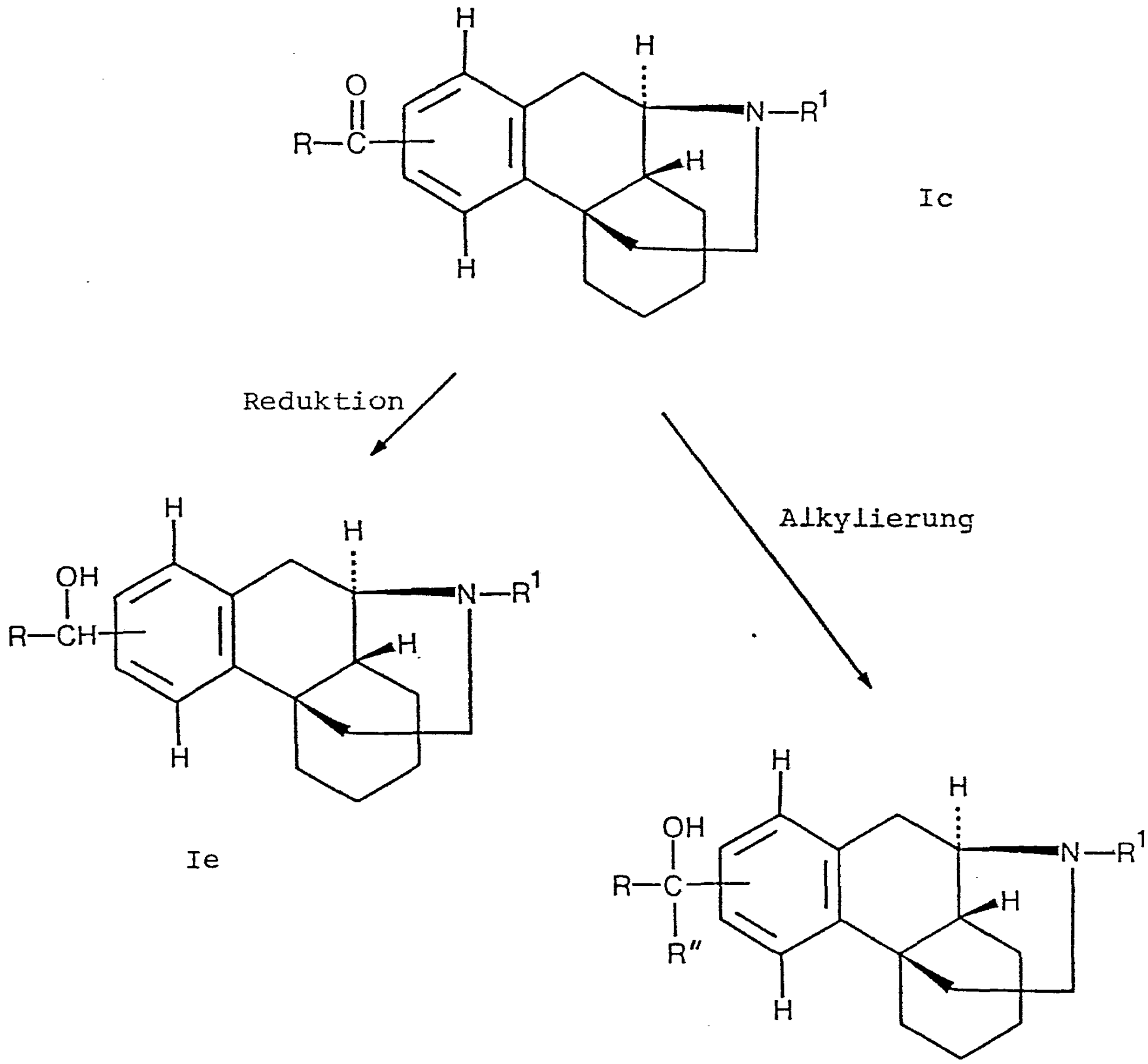


Ic

Ie

If

worin R" niederes Alkyl bedeutet und R und $R^1$ obige Bedeutung besitzen.

Die Verbindungen der obigen Formel I können sowohl mit anorganischen als auch mit organischen Säuren pharmazeutisch annehmbare Säureadditionssalze bilden. Die erfindungsgemässen Stoffe können demnach mit anorganischen Säuren, wie Salzsäure, Bromwasserstoff, Schwefelsäure und Phosphorsäure, und mit organischen Säuren, wie Weinsäure, Oxalsäure, Zitronensäure, Camphersulfonsäure, Aethansulfonsäure, Toluolsulfonsäure, Salicylsäure, Ascorbinsäure, Maleinsäure, Bernsteinsäure, Ameisensäure, Essigsäure und dergleichen, pharmazeutisch annehmbare Säureadditionssalze bilden.

Gegenstand der vorliegenden Erfindung sind: Morphinan-Derivate der Formel I und ihre pharmazeutisch annehmbaren Säureadditionssalze als solche und als pharmazeutische Wirkstoffe, insbesondere als analgetische Wirkstoffe; die Herstellung dieser Verbindungen; Zwischenprodukte für deren Herstellung, insbesondere die Verbindungen der Formeln III, IV, V und VI; Arzneimittel, enthaltend eine Verbindung der Formel I oder ein pharmazeutisch annehmbares Säureadditionssalz davon und die Herstellung solcher Arzneimittel; sowie die Verwendung der erfindungsgemässen Stoffe bei der Bekämpfung oder Verhütung von Krankheiten, insbesondere bei der Bekämpfung oder Verhütung von Schmerzen.

Wie bereits früher erwähnt, können die Verbindungen der Formel I und ihre pharmazeutisch annehmbaren Säureadditionssalze als Analgetika verwendet werden. Verabreicht man diese Stoffe oral oder parenteral, so entfalten sie auf die gleiche Weise wie Codein eine schmerzlindernde Wirkung.

Die Verbindungen der Formel I und ihre Salze können in herkömmliche pharmazeutische Dosierungsformen gebracht werden, und zwar mit den üblichen, für die orale oder parenterale Anwendung geeigneten pharmazeutischen Hilfsstoffen, z.B. mit inerten organischen oder anorganischen Trägern, wie Wasser, Gelatine, Lactose, Stärke, Magnesiumstearat, Talk, pflanzliche Oele, Gummi arabicum, Polyalkylenglykole und dergleichen. Die pharmazeutischen Präparate können als feste Formen vorliegen, z.B. als Tabletten, Suppositorien oder Kapseln, oder in flüssiger Form, z.B. als Lösungen, Suspensionen oder Emulsionen. Sie können auch pharmazeutische Hilfsstoffe, wie Konservierungsmittel, Stabilisierungsmittel, Netz- oder Emulgiermittel oder Salze, welche den osmotischen Druck verändern oder als Puffer wirken, enthalten. Die pharmazeutischen Präparate können auch noch andere therapeutisch wirksame Stoffe enthalten.

Die tägliche Dosierung der Verbindungen der Formel I variiert je nach Wirkungsstärke der verabreichten Verbindung, Art der Verabreichung und Gewicht des Patienten. Die tägliche Dosierung kann nicht durch definierte Grenzen festgesetzt werden; sie hängt vielmehr von der pharmakologischen Funktion der zu verabreichenden Verbindung der Formel I ab. Die orale Verabreichung ist repräsentativ für eine typische Methode, die Verbindungen der Formel I zu verabreichen. Bei der oralen Verabreichung von Tabletten dürfte eine tägliche Dosierung von 0,01 mg bis 0,15 mg/kg angemessen sein.

Wie bereits weiter oben erwähnt, werden die Verbindungen der Formel I und ihre Salze als schmerzstillende Mittel verwendet. Die analgetischen Eigenschaften können im Standard-"Phenylquinon writhing"-Test gezeigt werden (Sigmund et al., Proc. Soc. Exp. Biol. Med. 95, 729 [1957]). Die Verbindungen der Formel I gemäss der vorliegenden Erfindung lindern in signifikanter Weise die Schmerzen, welche in Mäusen chemisch-intraabdominal erzeugt wurden. Die $ED_{50}$ ist diejenige Dosis, welche die Gesamtzahl der "writhes" um 50% reduziert.

Die analgetischen Eigenschaften können auch im "tail flick"-Test gezeigt werden. Um die "tail flick"-Reaktionszeit nach oraler oder subkutaner Verabreichung einer erfindungsgemässen Verbindung der Formel I zu ermitteln, wurde die von Dewey und Harris beschriebene modifizierte Version des "tail flick"-Tests verwendet (S. Ehrenpreis und A. Neidle (Ed.), Methods in Narcotics Research, Marcel Dekker, Inc., New York, 1975, S. 101-108).

Gruppen von 10 männlichen, 18-22 g schweren Mäusen wurden einzeln in belüfteten, zylindrischen Käfigen aus rostfreiem Stahl plaziert, wobei ihre Schwänze jeweils durch ein Loch am einen Ende herausragen. Die Käfige wurden auf einer gleitenden Plattform befestigt, sodass jede Maus einer Lampe von hoher Intensität ausgesetzt werden konnte. Die Lichtstrahlen wurden mittels eines Parabolreflektors fokussiert und direkt auf den Schwanz gerichtet. Die Wärmeintensität wurde mit einem Rheostaten so justiert, dass unbehandelte Mäuse in etwa 2 bis 4 Sekunden reagieren. Es wurde die Zeit bis zum Auftreten der Reaktion (ein plötzliches Zucken des Schwanzes, um ihn aus dem heissen Lichtstrahl zu entfernen) ermittelt. Für den Fall, dass keine Reaktion innerhalb von 10 Sekunden ("cutoff time") beobachtet werden konnte, wurde der Versuch abgebrochen und eine Latenzzeit von 10 Sekunden für diesen Versuch notiert. Die Reaktionszeit für jede Maus wurde vor der Behandlung und jeweils 30, 60, 90 und 120 Minuten nach der Behandlung ermittelt.

Jede Gruppe von 10 Mäusen wurde oral oder subkutan mit einem Vehikel (normale Kochsalzlösung) oder mit logarythmisch festgesetzten Dosierungen einer Testverbindung behandelt. Für die Mehrzahl der Verbindungen waren vier Dosierungen pro Versuchsperiode genügend, um eine befriedigende Schätzung der $ED_{50}$ zu erlauben. Gelegentlich waren fünf oder sechs Dosierungen notwendig. In einigen Fällen wurden die Daten für eine gegebene Verbindung in zwei Versuchsperioden ermittelt, worauf die Resultate für die Analyse kombiniert wurden. Deshalb bestanden einige Kontrollgruppe und einige Testgruppen

aus insgesamt 20 Mäusen anstelle von 10.

Die berechneten Dosierungen ($ED_{50}$) beziehen sich auf die jeweils eingesetzten Salze. Das Injektionsvolumen betrug 0,1 ml/10 g Körpergewicht. Für jede Testverbindung wurden die $ED_{50}$-Werte für jeden Test separat berechnet. Für die Ermittlung der $ED_{50}$-Werte und ihrer 95-proz. Vertrauensbereiche wurden die Regressionsanalyse und Fieller's Theorem verwendet. Die Analyse wurde aufgrund der "tail flick"-Latenzzeit aller Mäuse ermittelt, welche mit einer Testverbindung behandelt worden sind. Für jeden Test wurde die durchschnittliche "tail flick"-Latenzzeit für jede Kontrollgruppe berechnet und daraus ein Latenzwert Z wie folgt abgeleitet:

$$Z = \frac{\text{"cutoff time" - durchschnittliche Latenzzeit Kontrollgruppe}}{2}$$

wobei die "cutoff time" 10 Sekunden betrug. Z repräsentierte somit 50% der maximal beobachtbaren Reaktionszeit, ausgehend von der durchschnittlichen Latenzzeit der Kontrollgruppe. Der Wert von Z wurde in der Regressionsanalyse als Interpoliergrösse verwendet, sodass die $ED_{50}$ diejenige Dosis angibt, welche die "tail flick"-Reaktionszeit um 50% der maximal beobachtbaren Reaktionszeit erhöht.

In der nachfolgenden Tabelle sind für alle getesteten Verbindungen der Formel I die ermittelten $ED_{50}$-Werte angegeben:

Tabelle I

| Verbindung | ED$_{50}$ (mg/kg) "Writhing"-Test Oral | Subku. | "Tail Flick"-Test Oral | Subku. |
|---|---|---|---|---|
| (-)-3-Acetyl-N-methyl-morphinan-D-tartrat | 0,50 | 0,08 | 2,0 | 3,9 |
| (-)-3-Acetyl-N-(cyclopropylmethyl)-morphinan-hydrochlorid | 2,1 | 0,18 | 1,5 | 2,3 |
| (-)-3-(1-Hydroxyäthyl)-N-methyl-morphinan-hydrochlorid | 0,36 | 0,24 | 1,1 | 1,3 |
| (-)-3-Acetyl-N-(2-phenäthyl)-morphinan-D-tartrat-hemihydrat | 1,2 | 0,39 | 10,4 | 1,8 |
| (-)-3-Propionyl-N-methylmorphinan-dihydrochlorid | 1,9 | 0,22 | 12,0 | 2,3 |

Die folgenden Beispiele dienen der näheren Erläuterung der vorliegenden Erfindung. Alle Temperaturen sind in Grad Celsius angegeben.

## Beispiel 1

a) Man versetzt eine unter Stickstoff und bei Eisbad-Temperatur rasch gerührte Mischung von 44,0 g wasserfreiem Aluminiumchlorid und 450 ml 1,2-Dichloräthan über einen Zeitraum von 15 Minuten portionenweise mit insgesamt 37,0 g (-)-N-Methylmorphinan-hydrochlorid, rührt noch während 15 Minuten bei dieser Temperatur, versetzt dann tropfenweise mit einer Lösung von 20,12 g frisch destilliertem Acetylchlorid in 15 ml 1,2-Dichloräthan, entfernt nach beendeter Zugabe das Kühlbad und lässt die Mischung auf Raumtemperatur erwärmen. Man erhitzt anschliessend während 3 Stunden unter Rückfluss zum Sieden, kühlt ab und giesst auf 800 ml Wasser. Die wässrige Suspension wird mit 10N-Natronlauge basisch gestellt und zweimal mit je 1 l Chloroform extrahiert. Die vereinigten Auszüge werden mit 1 l Wasser gewaschen, über Magnesiumsulfat getrocknet und eingedampft. Man erhält 26,9 g (71%) einer rohen Mischung, bestehend aus (-)-3-Acetyl-N-methylmorphinan und (-)-2-Acetyl-N-methylmorphinan.

b) Man vereinigt eine heisse Lösung von 63,4 g einer Mischung von (-)-3- und (-)-2-Acetyl-N-methylmorphinan und 238 ml Aethanol mit einer heissen Lösung von 34,0 g d-Weinsäure in 238 ml Aethanol, impft die klare Lösung mit einigen wenigen Kristallen von (-)-3-Acetyl-N-methylmorphinan d-tartrat an und lässt während 24 Stunden bei Raumtemperatur kristallisieren. Die erhaltene Kristalle werden dann abfiltriert, mit Aethanol gewaschen und aus 600 ml heissem Aethanol umkristallisiert (während 24 Stunden bei Raumtemperatur). Man erhält 34,6 g (36%) reines (-)-3-Acetyl-N-methylmorphinan d-tartrat vom Schmelzpunkt 179-181°; $[\alpha]_D^{25}$ = -6,10° (c = 1,17 in Methanol).

c) Man suspendiert 4,30 g (-)-3-Acetyl-N-methylmorphinan d-tartrat in 20 ml Wasser, stellt mit konzentriertem Ammoniak basisch und extrahiert zweimal mit je 40 ml

Aether. Man wäscht die Aetherauszüge mit Wasser und trocknet sie über Magnesiumsulfat. Nach Entfernen des Lösungsmittels erhält man 2,8 g (99%) (-)-3-Acetyl-N-methylmorphinan. Für die Analyse wurde eine Probe dieser Substanz destilliert: Siedepunkt = 175-185° (0,2 mm), $[\alpha]_D^{25}$ = -47,14° (c = 1,17 in Methanol).

Das Perchloratsalz von (-)-3-Acetyl-N-methylmorphinan wird mit 70-proz. Perchlorsäure in Aethanol hergestellt; Schmelzpunkt = 226-228°, $[\alpha]_D^{25}$ = -20,64° (c = 1,01 in Methanol).

## Beispiel 2

a) Die bei der Abtrennung von (-)-3-Acetyl-N-methylmorphinan d-tartrat in Beispiel 1b) erhaltenen Filtrate werden auf 400 ml eingeengt und dann auf dem Dampfbad erwärmt, bis man eine klare Lösung erhält. Die Lösung wird mit einigen wenigen Kristallen von (-)-2-Acetyl-N-methylmorphinan d-tartrat angeimpft und zum Kristallisieren während 7 Tagen bei Raumtemperatur stehen gelassen. Die Kristalle werden abfiltriert, mit Aethanol gewaschen und aus insgesamt 450 ml heissem Aethanol umkristallisiert (während 7 Tagen bei Raumtemperatur). Die Kristalle werden abfiltriert, mit Aethanol gewaschen und getrocknet, wobei man 18,0 g (19%) rohes (-)-2-Acetyl-N-methylmorphinan d-tartrat vom Schmelzpunkt 168-170° erhält. Eine dritte und letzte Umkristallisation aus insgesamt 300 ml Aethanol während 21 Tagen bei Raumtemperatur liefert 16,3 g (17%) reines (-)-2-Acetyl-N-methylmorphinan d-tartrat vom Schmelzpunkt 188-190°; $[\alpha]_D^{25}$ = -4,90° (c = 1,01 in Methanol).

b) Man suspendiert 0,25 g (-)-2-Acetyl-N-methylmorphinan d-tartrat in Wasser und stellt mit konzentriertem Ammoniak basisch. Man extrahiert die erhaltene Suspension mit Aether, wäscht die vereinigten Auszüge mit Wasser und trocknet diese über Magnesiumsulfat. Nach Entfernen des

Lösungsmittels erhält man rohes (-)-2-Acetyl-N-methylmorphinan. Für die Analyse wird dieser Stoff destilliert: Siedepunkt = 175-180° (0,1 mm), $[\alpha]_D^{25}$ = -55,67° (c = 1,04 in Methanol).

Beispiel 3

a) Man versetzt eine Mischung von 2,7 g (-)-3-Acetyl-N-methylmorphinan, 50 ml Benzol und 20 mg Kaliumcarbonat tropfenweise mit 2,11 g 2,2,2-Trichloräthylchloroformat in 10 ml Benzol. Man rührt die Reaktionsmischung während 48 Stunden bei der Rückflusstemperatur, kühlt ab und verdünnt mit 100 ml Aether. Man extrahiert zweimal mit je 30 ml 1N-Salzsäure und trocknet die organische Phase über Magnesiumsulfat. Nach Entfernen des Lösungsmittels erhält man 2,8 g (66%) rohes (-)-3-Acetylmorphinan-17-carbonsäure-(trichloräthyl)ester. Zur Analyse wird eine Probe dieser Substanz destilliert: Siedepunkt = 230-240° (0,1 mm), $[\alpha]_D^{25}$ = -127,21° (c = 1,11 in Methanol).

b) Man versetzt eine Lösung von 2,4 g (-)-3-Acetylmorphinan-17-carbonsäure-(trichloräthyl)ester in 40 ml 90-proz. Essigsäure portionenweise mit 2,5 g Zinkstaub, rührt die erhaltene Mischung während 16 Stunden bei Raumtemperatur und filtriert. Das Filtrat wird im Vakuum eingedampft, worauf man den Rückstand zwischen 40 ml Aether und verdünntem Ammoniak verteilt. Man extrahiert die ätherische Lösung mit 60 ml 4N-Salzsäure, stellt die saure Lösung mit konzentriertem Ammoniak basisch und extrahiert zweimal mit je 30 ml Aether. Die Aetherauszüge werden über Magnesiumsulfat getrocknet und eingedampft, wobei man 1,0 g (71%) rohes (-)-3-Acetylmorphinan erhält. Zur Analyse wird eine Probe dieses Stoffes destilliert; Siedepunkt = 165-175° (0,1 mm), $[\alpha]_D^{25}$ = -24,77° (c = 1,06 in Methanol).

## Beispiel 4

Man versetzt eine Mischung von 0,8 g (-)-3-Acetyl-morphinan, 0,6 g Natriumbicarbonat und 10 ml Dimethylformamid mit 0,3 g Cyclopropylmethylchlorid, erhitzt während 18 Stunden unter Rückfluss zum Sieden, kühlt auf Raumtemperatur ab und filtriert. Das Filtrat wird unter vermindertem Druck eingedampft, worauf man den Rückstand in 60 ml Aether aufnimmt, die ätherische Lösung mit Wasser wäscht, über Magnesiumsulfat trocknet und eindampft. Man erhält 0,8 g (83%) rohes (-)-3-Acetyl-N-(cyclopropymethyl)morphinan. Zur Analyse wird eine Probe dieses Stoffes destilliert: Siedepunkt = 185-195° (0,1 mm), $[\alpha]_D^{25}$ = -83,21° (c = 1,02 in Methanol).

Durch Behandeln von 0,69 g der obigen Base mit wasserfreiem Chlorwasserstoff in Essigester erhält man nach Umkristallisieren aus Aceton/Aether 0,7 g (91%) (-)-3-Acetyl-N-(cyclopropylmethyl)morphinan-hydrochlorid vom Schmelzpunkt 263-265°; $[\alpha]_D^{25}$ = -57,25° (c = 0,98 in Methanol).

## Beispiel 5

Man versetzt eine Mischung von 1,7 g (-)-3-Acetyl-morphinan, 1,5 g Kaliumcarbonat und 40 ml Dimethylformamid mit 1,2 g Cyclobutylmethylchlorid, erhitzt während 16 Stunden auf 100-110°, kühlt auf Raumtemperatur ab und filtriert. Man dampft das Filtrat unter vermindertem Druck ein, nimmt den Rückstand in 120 ml Aether auf, wäscht die ätherische Lösung zweimal mit je 20 ml Wasser, trocknet sie über Magnesiumsulfat und dampft ein. Man erhält 1,6 g (76%) rohes (-)-3-Acetyl-N-(cyclobutylmethyl)mor-phinan. Zur Analyse wird eine Probe dieser Verbindung destilliert: Siedepunkt = 200-210° (0,05 mm), $[\alpha]_D^{25}$ = -80,64° (c = 1,03 in Methanol).

Durch Behandeln von 1,3 g der rohen Base mit wasserfreiem Chlorwasserstoff in Essigester erhält man das rohe Hydrochlorid, woraus man nach Kristallisieren aus Aethanol/Aether 0,186 g (13%) (-)-3-Acetyl-N-(cyclobutylmethyl)morphinan-hydrochlorid vom Schmelzpunkt 220-222° erhält; $[\alpha]_D^{25}$ = -54,43° (c = 0,81 in Methanol).

Beispiel 6

Man erhitzt eine Mischung von 1,5 g (-)-3-Acetylmorphinan, 1,3 g (2-Bromäthyl)benzol und 1,1 g wasserfreiem Kaliumcarbonat in 40 ml Dimethylformamid während 16 Stunden unter Stickstoff bei 110°, kühlt ab, filtriert die Reaktionsmischung und dampft das Filtrat unter vermindertem Druck ein. Den Rückstand nimmt man in Aether auf, wäscht mit Wasser und trocknet über Magnesiumsulfat. Nach Entfernen des Lösungsmittels erhält man 1,8 g (85%) rohes (-)-3-Acetyl-N-(2-phenäthyl)morphinan. Zur Analyse wird eine Probe dieses Stoffes destilliert: Siedepunkt 210-220° (0,05 mm), $[\alpha]_D^{25}$ = -100,72° (c = 1,08 in Methanol).

Man löst 1,7 g der obigen Base in 3 ml heissem Aethanol und vereinigt diese Lösung mit einer heissen Lösung von 0,72 g d-Weinsäure in 4 ml Aethanol. Man lässt während 2 Stunden bei Raumtemperatur kristallisieren und filtriert die Kristalle ab. Man erhält 2,2 g (92%) (-)-3-Acetyl-N-(2-phenäthyl)morphinan d-tartrat-hemihydrat vom Schmelzpunkt 100-102° (Zersetzung); $[\alpha]_D^{25}$ = -45,69° (c = 1,08 in Methanol).

Beispiel 7

Man versetzt eine Mischung von 1,5 g (-)-3-Acetylmorphinan, 2,3 g Triäthylamin und 35 ml trockenem Aethanol mit 0,35 g Acrylnitril, erhitzt die Mischung während 16 Stunden unter Rückfluss zum Sieden und entfernt das Lösungsmittel sowie überschüssiges Reagens unter vermindertem Druck. Man erhält 1,7 g (94%) rohes (-)-3-Acetyl-N-(2-cyanoäthyl)morphinan. Zur Analyse wird eine Probe dieser Verbindung destilliert: Siedepunkt = 220-230° (0,05 mm), $[\alpha]_D^{25}$ = -65,5° (c = 1,03 in Methanol).

Durch Behandeln von 1,6 g der obigen Base mit wasserfreiem Chlorwasserstoff in Essigester erhält man das rohe Hydrochlorid, woraus man durch Umkristallisieren aus Aethanol/Aether 1,6 g (94%) (-)-3-Acetyl-N-(2-cyanoäthyl)morphinan-hydrochlorid vom Schmelzpunkt 245-247° (Zersetzung) erhält; $[\alpha]_D^{25}$ = -54,93° (c = 0,94 in Methanol).

Beispiel 8

a) Man versetzt eine Mischung von 1,5 g (-)-3-Acetylmorphinan, 1,7 g Triäthylamin und 15 ml Methylenchlorid bei Eisbad-Temperatur und über einen Zeitraum von 1 Stunde mit einer Lösung von 1,3 g 2-Furylacetylchlorid in 10 ml Methylenchlorid, erhitzt während 4 Stunden unter Rückfluss zum Sieden, kühlt auf Raumtemperatur ab und entfernt das Lösungsmittel unter vermindertem Druck. Man nimmt den Rückstand in 100 ml Aether auf, wäscht nacheinander zweimal mit je 15 ml 1N-Salzsäure und Wasser, trocknet über Magnesiumsulfat und entfernt das Lösungsmittel. Man erhält 1,9 g (90%) rohes (-)-3-Acetyl-N-[(2-furylmethyl)carbonyl]morphinan. Zur Analyse wird eine Probe dieses Stoffes destilliert: Siedepunkt = 235-245° (0,05 mm), $[\alpha]_D^{25}$ = -160,43° (c = 0,94 in Methanol).

b) Man erhitzt eine Mischung von 1,2 g (-)-3-Acetyl-N-[(2-furylmethyl)carbonyl]morphinan, 0,5 g Aethylenglykol und 20 ml Benzol, das eine katalytische Menge p-Toluolsulfonsäure enthält, während 20 Stunden unter Rückfluss zum Sieden, wobei man das gebildete Reaktionswasser mittels eines Dean-Stark-Apparates entfernt. Nach Abkühlen wird die Lösung nacheinander mit 20 ml 1N-Natronlauge und zweimal mit je 20 ml Wasser gewaschen, über Magnesiumsulfat getrocknet und unter vermindertem Druck

eingedampft. Man erhält 1,1 g (84%) des entsprechenden Ketals, das ohne weitere Reinigung in die nächste Stufe eingesetzt wird. Man versetzt eine Suspension von 55 mg Lithiumaluminiumhydrid in 8 ml Tetrahydrofuran tropfenweise mit 0,5 g des obigen Ketals in 6 ml Tetrahydrofuran, erhitzt über Nacht unter Rückfluss zum Sieden, kühlt auf Raumtemperatur ab und versetzt tropfenweise mit 5 ml Essigsäure und anschliessend 2 ml Wasser. Man trennt die organische Phase ab, entfernt das Lösungsmittel unter vermindertem Druck und verteilt den Rückstand zwischen 25 ml 1N-Salzsäure und 25 ml Aether. Die saure Lösung wird mit konzentriertem Ammoniak basisch gestellt und zweimal mit je 25 ml Aether extrahiert. Die vereinigten Aetherauszüge werden zweimal mit je 20 ml Wasser gewaschen, über Magnesiumsulfat getrocknet und eingedampft. Man erhält 0,21 g (49%) rohes (-)-3-Acetyl-N-[2-(2-furyl)äthyl]morphinan. Diese Verbindung chromatographiert man an 50 g Kieselgel, wobei man mit 6-proz. Methanol in Methylenchlorid eluiert, und erhält 0,16 g (37%) reines (-)-3-Acetyl-N-[2-(2-furyl)äthyl]morphinan vom Siedepunkt 185-190° (0,06 mm); $[\alpha]_D^{25} = -97,91°$ (c = 0,91 in Methanol).

Durch Behandeln von 0,16 g der obigen Base mit wasserfreiem Chlorwasserstoff in Essigester erhält man das rohe Hydrochlorid, woraus man nach Umkristallisieren aus Acetonitril/Aether reines (-)-3-Acetyl-N-[2-(2-furyl)-äthyl]morphinan-hydrochlorid vom Schmelzpunkt 205-207° erhält; $[\alpha]_D^{25} = -67,45°$ (c = 0,85 in Methanol).

## Beispiel 9

Man versetzt eine Lösung von 3,0 g (-)-3-Acetyl-N-methylmorphinan in 50 ml Methanol unter Rühren portionenweise mit insgesamt 3,0 g Natriumborhydrid, rührt die Mischung während 16 Stunden bei Raumtemperatur, entfernt das Lösungsmittel unter vermindertem Druck und verteilt den Rückstand zwischen 50 ml Wasser und 100 ml Aether.

Man wäscht die Aetherlösung mit Wasser, trocknet sie über Magnesiumsulfat und entfernt das Lösungsmittel, wobei man 2,8 g (93%) rohes (-)-3-(1-Hydroxyäthyl)-N-methylmorphinan als Epimerengemisch erhält. Zur Analyse wird eine Probe dieser Verbindung destilliert: Siedepunkt = 170-180° (0,05 mm), $[\alpha]_D^{25}$ = -41,06° (c = 1,07 in Methanol).

Durch Behandeln von 2,8 g der rohen Base mit wasserfreiem Chlorwasserstoff in Essigester erhält man das rohe Hydrochlorid, woraus man durch Kristallisieren aus Aethanol/Aether 2,5 g (79%) reines (-)-3-(1-Hydroxyäthyl)-N-methylmorphinan-hydrochlorid vom Schmelzpunkt 219-221° erhält, $[\alpha]_D^{25}$ = -30,0° (c = 1,04 in Methanol).

## Beispiel 10

Man versetzt eine unter Stockstoff gerührte Lösung von 2,0 g (-)-3-Acetyl-N-methylmorphinan in 100 ml Aether unter Kühlen in einem Eisbad und über einen Zeitraum von 20 Minuten tropfenweise mit insgesamt 17,0 ml Methyllithium (1,3M in Aether). Man lässt auf Raumtemperatur erwärmen, rührt noch während 20 Minuten bei dieser Temperatur, giesst auf Eiswasser und trennt die ätherische Phase ab. Die wässrige Lösung wird noch zweimal mit je 50 ml Aether extrahiert, worauf man die vereinigten ätherischen Auszüge mit Wasser wäscht, über Magnesiumsulfat trocknet und eindampft. Man erhält 1,7 g (81%) (-)-3-(2-Hydroxy-2-propyl)-N-methylmorphinan. Zur Analyse wird eine Probe dieses Stoffes destilliert: Siedepunkt = 175-180° (0,05 mm), $[\alpha]_D^{25}$ = -38,6° (c = 1,09 in Methanol).

Durch Behandeln von 1,7 g der rohe Base mit wasserfreiem Chlorwasserstoff in Essigester erhält man das rohe Hydrochlorid, woraus man durch Umkristallisieren aus Aceton 1,9 g (99%) reines (-)-3-(2-Hydroxy-2-propyl)-N-methylmorphinan-hydrochlorid vom Schmelzpunkt 206-208° erhält; $[\alpha]_D^{25}$ = -21,95° (c = 0,97 in Methanol).

Beispiel 11

Man versetzt eine unter Stickstoff und bei Eisbad-Temperatur stark gerührte Mischung von 14,2 g wasserfreiem Aluminiumchlorid und 140 ml 1,2-Dichloräthan über einen Zeitraum von 10 Minuten portionenweise mit insgesamt 12,0 g (-)-N-Methylmorphinan-hydrochlorid. Man rührt noch während 15 Minuten bei dieser Temperatur, versetzt tropfenweise mit 7,6 g Propionylchlorid in 5 ml 1,2-Dichloräthan, entfernt nach beendeter Zugabe das Kühlbad und lässt auf Raumtemperatur erwärmen. Man erhitzt dann während 3 Stunden unter Rückfluss zum Sieden, kühlt ab und giesst auf 500 ml Eiswasser. Die wässrige Suspension wird mit konzentriertem Ammoniak basisch gestellt und zweimal mit je 250 ml Methylenchlorid extrahiert. Die vereinigten Auszüge werden mit 300 ml Wasser gewaschen, über Magnesiumsulfat getrocknet und eingedampft. Man erhält 11,8 g (92%) einer rohen Mischung von (-)-3-Propionyl-N-methylmorphinan und (-)-2-Propionyl-N-methylmorphinan.

11,8 g der obigen rohen Mischung liefern nach Behandeln mit wasserfreiem Chlorwasserstoff in Aceton das rohe Hydrochlorid. Man löst die rohe Mischung in 40 ml heissem Aethanol und lässt über Nacht bei Raumtemperatur kristallisieren. Man erhält 4,7 g (36%) (-)-3-Propionyl-N-methylmorphinan-hydrochlorid vom Schmelzpunkt 278-280°, $[\alpha]_D^{50}$ = -29,51° (c = 1,13 in Methanol).

Zur Analyse stellt man aus dem obigen Hydrochlorid die freie Base her, wobei man als Base Ammoniak verwendet und mit Methylenchlorid extrahiert. Das erhaltene (-)-3-Propionyl-N-methylmorphinan wird destilliert: Siedepunkt = 185-190° (0,025 mm), $[\alpha]_D^{25}$ = -47,60° (c = 1,05 in Methanol).

Beispiel 12

a) Man versetzt eine unter Stickstoff rasch gerührte Mischung von 5,8 g wasserfreiem Aluminiumchlorid und 65 ml über Aluminiumoxid der Aktivitätsstufe I getrocknetem 1,2-Dichloräthan bei Eisbadtemperatur portionenweise mit insgesamt 6,2 g (-)-N-Methylmorphinan-hydrochlorid. Man rührt die Reaktionsmischung noch während 15 Minuten und versetzt über einen Zeitraum von 40 Minuten tropfenweise mit einer Lösung von 6,3 g destilliertem Benzoylchlorid in 10 ml 1,2-Dichloräthan. Nach beendeter Zugabe entfernt man das Kühlbad und lässt die Mischung unter Rühren auf Raumtemperatur erwärmen. Man erhitzt noch während 16,5 Stunden unter Rückfluss zum Sieden, kühlt ab und giesst auf 100 ml Eiswasser. Man wäscht die wässrige Mischung mit 100 ml Aether, stellt sie mit 50 ml 10N-Natronlauge basisch und extrahiert dreimal mit je 100 ml Methylenchlorid. Die vereinigten Methylenchloridauszüge werden mit 100 ml Wasser gewaschen, über Magnesiumsulfat getrocknet und eingedampft. Man erhält 6,7 g (87%) einer rohen Mischung von (-)-3-Benzoyl-N-methylmorphinan und (-)-2-Benzoyl-N-methylmorphinan.

b) Man vereinigt eine heisse Lösung von 6,7 g der obigen rohen Mischung in 75 ml Aethanol mit einer heissen Lösung von 2,95 g d-Weinsäure in 25 ml Aethanol, lässt während 6 Tagen bei Raumtemperatur kristallisieren und filtriert die erhaltenen Kristalle ab. Das kristalline Material wird mit Aethanol gewaschen und aus 50 ml Methanol während 6 Tagen bei Raumtemperatur umkristallisiert, wobei man 2,5 g (26%) reines (-)-3-Benzoyl-N-methylmorphinan d-tartrat vom Schmelzpunkt 203-204° erhält; $[\alpha]_D^{25}$ = -33,36° (c = 9,92 in Methanol).

Man suspendiert 0,5 g des obigen Salzes in Wasser, stellt mit 5-proz. Natronlauge basisch und extrahiert die erhaltene Suspension mit Methylenchlorid. Die vereinigten Auszüge werden mit Wasser gewaschen und über Magnesiumsulfat getrocknet. Nach Entfernen des Lösungsmittels erhält man (-)-3-Benzoyl-N-methylmorphinan, welches destilliert wird: Siedepunkt 220-230° (0,025 mm), $[\alpha]_D^{25}$ = -77,07° (c = 0,91 in Methanol). Eine Probe dieses Stoffes wurde auch aus Aether kristallisiert: Schmelzpunkt 103-104°.

## Beispiel 13

Man versetzt eine Mischung von 1,8 g (-)-3-Acetylmorphinan, 0,6 g Natriumbicarbonat und 25 ml Dimethylformamid mit 1,0 g 3,3-Dimethylalllylbromid, erwärmt während 16 Stunden auf 50°, kühlt die Mischung auf Raumtemperatur ab und filtriert. Man dampft das Filtrat unter vermindertem Druck ein und verteilt den Rückstand zwischen 50 ml Methylenchlorid und 30 ml Wasser. Die Methylenchloridlösung wird mit Wasser gewaschen, über Magnesiumsulfat getrocknet und eingedampft. Man erhält 2,1 g (93%) rohes (-)-3-Acetyl-N-(3-methyl-2-butenyl)-morphinan. Zur Analyse wird eine Probe dieser Verbindung destilliert: Siedepunkt 185-190° (0,05 mm), $[\alpha]_D^{25}$ = -76,53° (c = 0,98 in Methanol).

Man vereinigt eine heisse Lösung von 2,1 g der obigen Base in 7 ml Aceton mit einer heissen Lösung von 0,92 g d-Weinsäure in Aceton und lässt die Mischung bei Raumtemperatur kristallisieren. Aus dem rohen Tartrat erhält man nach Kristallisieren aus Isopropanol 1,7 g (55%) (-)-3-Acetyl-N-(3-methyl-2-butenyl)morphinan d-tartratmonohydrat vom Schmelzpunkt 105-110° (Zersetzung), $[\alpha]_D^{25}$ = -38,49° (c = 0,98 in Methanol).

## Beispiel A

Herstellung von Tabletten:

| | mg/Tablette |
|---|---|
| (-)-3-Acetyl-N-(cyclopropylmethyl)-morphinan-hydrochlorid | 5,0 |
| Lactose | 99,0 |
| Vorgelatinisierte Stärke | 10,0 |
| Kornstärke | 15,0 |
| Modifizierte Stärke | 10,0 |
| Magnesiumstearat | 1,0 |
| Tablettengewicht | 140 mg |

Man mischt den Wirkstoff, die Lactose, die vorgelatinisierte Stärke, die Kornstärke und die modifizierte Stärke in einem geeigneten Mixer, granuliert mit Wasser, trocknet über Nacht in einem Ofen, mahlt in einer geeigneten Mühle, vermischt mit dem Magnesiumstearat und verpresst auf einer geeigneten Presse zu Tabletten.

## Beispiel B

Herstellung von Tabletten:

| | mg/Tablette |
|---|---|
| (-)-3-Acetyl-N-(cyclopropylmethyl)-morphinan-hydrochlorid | 10,0 |
| Lactose | 103,0 |
| Avicel | 45,0 |
| Modifizierte Stärke | 10,0 |
| Kornstärke | 30,0 |
| Magnesiumstearat | 2,0 |
| Tablettengewicht | 200 mg |

Man mischt den Wirkstoff, die wasserfreie Lactose, das Avicel, die modifizierte Stärke und die Kornstärke in einem geeigneten Mixer während 10-15 Minuten, fügt das

Magnesiumstearat als Vormischung dazu, mischt während 4 Minuten und verpresst auf einer geeigneten Presse zu Tabletten.

Beispiel C

Herstellung von Kapseln:

| Bestandteile | mg/Kapsel |
|---|---|
| (-)-3-Acetyl-N-(cyclopropylmethyl)-morphinan-hydrochlorid | 10,0 |
| Lactose | 218,0 |
| Kornstärke | 50,0 |
| Mgnesiumstearat | 2,0 |
| Talk | 10,0 |
| Kapselfüllgewicht | 290 mg |

Man vermischt den Wirkstoff, die Lactose und die Kornstärke in einem geeigneten Mixer, vermahlt in einer geeigneten Mühle, vermischt mit dem Magnesiumstearat und dem Talk und füllt auf einer Kapselabfüllmaschine in Kapseln ab.

Beispiel D

Herstellung von Kapseln:

| Bestandteile | mg/Kapsel |
|---|---|
| (-)-3-Acetyl-N-methylmorphinan d-tartrat | 10,0 |
| Lactose | 218,0 |
| Kornstärke | 70,0 |
| Magnesiumstearat | 3,0 |
| Talk | 15,0 |
| Kapselfüllgewicht | 340 mg |

Man vermischt den Wirkstoff, die Lactose und die Kornstärke in einem geeigneten Mischer, mahlt in einer geeigneten Mühle, vermischt mit dem Magnesiumstearat und

dem Talk und füllt auf einer Kapselabfüllmaschine in Kapseln ab.

## Beispiel E

Herstellung von Tabletten (Nassgranulation):

| Bestandteile | mg/Tablette |
|---|---|
| (-)-3-Acetyl-N-methylmorphinan d-tartrat | 0,5 |
| Lactose | 186,5 |
| Modifizierte Stärke | 35 |
| Vorgelatinisierte Stärke | 24 |
| Destilliertes Wasser q.s. | - |
| Magnesiumstearat | 4 |
| Tablettengewicht | 250 mg |

Man vermischt den Wirkstoff, die Lactose, die modifizierte Stärke und die vorgelatinisierte Stärke in einem geeigneten Mischer, granuliert mit genügend destilliertem Wasser, um eine geeignete Konsistenz zu erhalten, mahlt, trocknet in einem geeigneten Ofen, mahlt und vermischt mit dem Magnesiumstearat während 3 Minuten und verpresst auf einer geeigneten Presse zu Tabletten geeignete Grösse.

## Beispiel F

Herstellung von Tabletten (Nassgranulation):

| Bestandteile | mg/Tablette |
|---|---|
| (-)-3-Acetyl-N-(cyclopropylmethyl)-morphinan-hydrochlorid | 2,0 |
| Lactose | 253,0 |
| Modifizierte Stärke | 55 |
| Vorgelatinisierte Stärke | 35 |
| Destilliertes Wasser q.s. | - |
| Magnesiumstearat | 5 |
| Tablettengewicht | 350 mg |

Man vermischt den Wirkstoff, die Lactose, die modifizierte Stärke und die vorgelatinisierte Stärke in einem geeigneten Mixer, granuliert mit genügend destilliertem Wasser, um eine geeignete Konsistenz zu erhalten, mahlt, trocknet in einem geeigneten Ofen, mahlt, vermischt während 3 Minuten mit dem Magnesiumstearat und verpresst auf einer geeigneten Presse zu Tabletten geeigneter Grösse.

## Patentansprüche

1. Morphinanderivate der allgemeinen Formel

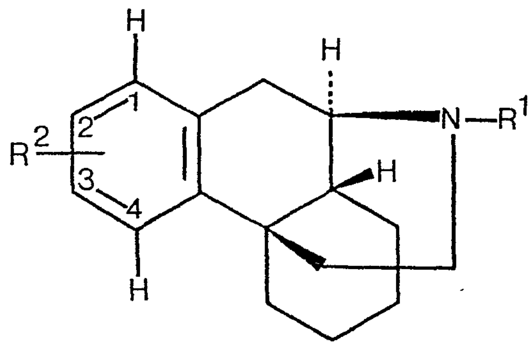

I

worin $R^1$ Wasserstoff, niederes Alkyl, niederes Alkenyl, niederes Cycloalkyl-niederes Alkyl, Cyano-niederes Alkyl, Aryl-niederes Alkyl oder Heteroaryl-niederes Alkyl, $R^2$ $(C_{2-7})$-Alkanoyl, Arylcarbonyl, Trifluormethylcarbonyl oder die Gruppe RR'C(OH)-, R $(C_{1-6})$-Alkyl und R' Wasserstoff oder niederes Alkyl bedeuten,
und pharmazeutisch annehmbare Säureadditionssalze davon.

2. Verbindungen gemäss Anspruch 1, worin $R^2$ sich in Stellung 3 befindet.

3. Verbindungen gemäss Anspruch 2, worin $R^2$ $(C_{2-7})$-Alkanoyl bedeutet.

4. Verbindungen gemäss Anspruch 2 oder 3, worin $R^1$ niederes Alkyl, niederes Alkenyl, niederes Cycloalkyl-niederes Alkyl oder Aryl-niederes Alkyl bedeutet.

5. Verbindungen gemäss Anspruch 2, worin $R^2$ die Gruppe RR'C(OH)-, R $(C_{1-6})$-Alkyl und R' Wasserstoff oder niederes Alkyl bedeuten.

6. Verbindungen gemäss Anspruch 5, worin $R^1$ niederes Alkyl bedeutet.

7. (-)-3-Acetyl-N-(cyclopropylmethyl)morphinan.

8. (-)-3-(1-Hydroxyäthyl)-N-methylmorphinan.

9. (-)-3-Acetyl-N-(cyclobutylmethyl)morphinan.

10. (-)-3-Acetyl-N-methylmorphinan.

11. (-)-3-Propionyl-N-methylmorphinan.

12. (-)-3-(2-Hydroxy-2-propyl)-N-methylmorphinan.

13. (-)-3-Acetyl-N-(3-methyl-2-butenyl)morphinan.

14. (-)-3-Benzoyl-N-methylmorphinan.

15. (-)-3-Acetyl-N-(2-phenäthyl)morphinan.

16. (-)-3-Acetyl-N-(2-cyanoäthyl)morphinan.

17. (-)-2-Acetyl-N-methylmorphinan.

18. Verbindungen der allgemeinen Formel

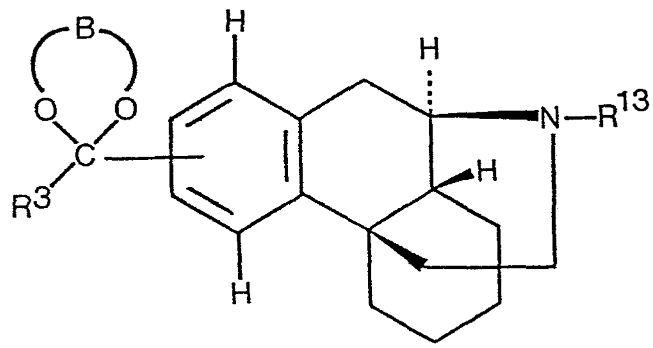

III

worin $R^{13}$ niederes Alkyl, niederes Cyloalkyl-niederes Alkyl, Aryl-niederes Alkyl oder Heteroaryl-niederes Alkyl, B Dimethylen oder Trimethylen und $R^3$ $(C_{1-6})$-Alkyl, Aryl oder Trifluormethyl bedeuten.

19. Verbindungen der allgemeinen Formel

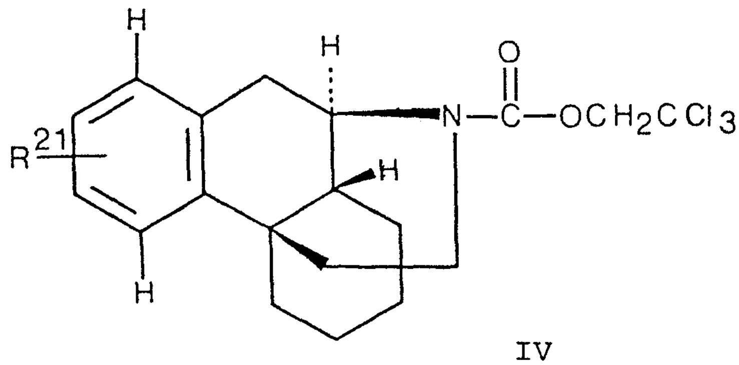

IV

worin $R^{21}$ $(C_{2-7})$-Alkanoyl, Arylcarbonyl oder Trifluormethylcarbonyl bedeutet.

20. Verbindungen der allgemeinen Formel

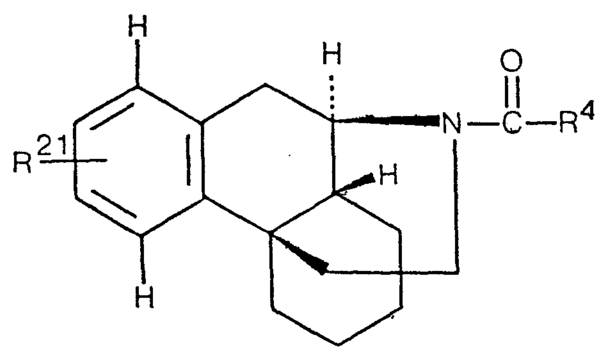

V

worin $R^{21}$ $(C_{2-7})$-Alkanoyl, Arylcarbonyl oder Trifluormethylcarbonyl und $R^4$ Wasserstoff, $(C_{1-6})$-Alkyl, niederes Cycloalkyl, niederes Cycloalkyl-$(C_{1-6})$-Alkyl, Aryl, Aryl-$(C_{1-6})$-Alkyl, Heteroaryl oder Heteroaryl-$(C_{1-6})$-alkyl bedeuten.

21. Verbindungen der allgemeinen Formel

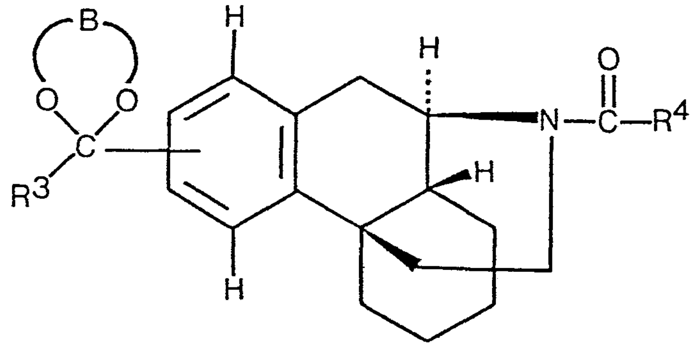

VI

worin B Dimethylen oder Trimethylen, $R^3$ $(C_{1-6})$-Alkyl, Aryl oder Trifluormethyl und $R^4$ Wasserstoff, $(C_{1-6})$-Alkyl, niederes Cycloalkyl, niederes Cyclo-alkyl-$(C_{1-6})$-alkyl, Aryl, Aryl-$(C_{1-6})$-alkyl, Heteroaryl oder Heteroaryl-$(C_{1-6})$-alkyl bedeuten.

22. Verbindungen gemäss einem der Ansprüche 1-17 als pharmazeutische Wirkstoffe.

23. Verbindungen gemäss einem der Ansprüche 1-17 als analgetische Wirkstoffe.

24. Verfahren zur Herstellung von Verbindungen gemäss einem der Ansprüche 1-17, dadurch gekennzeichnet, dass man

a) eine Verbindung der allgemeinen Formel

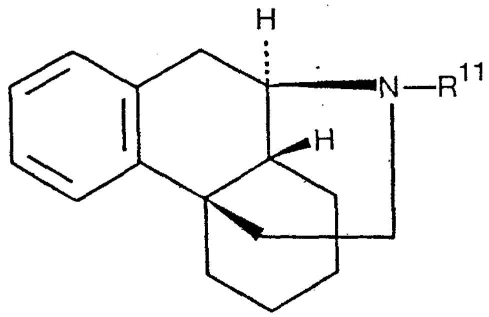

II

worin $R^{11}$ niederes Alkyl bedeutet, acyliert, oder

b) eine Verbindung der allgemeinen Formel

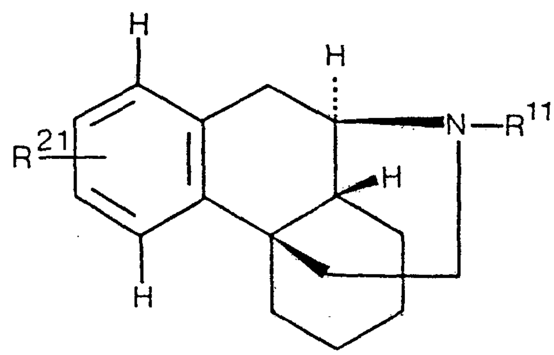

Ia

worin $R^{21}$ $(C_{2-7})$-Alkanoyl, Arylcarbonyl oder Trifluormethylcarbonyl bedeutet und $R^{11}$ obige Bedeutung besitzt, N-dealkyliert, oder

c) eine Verbindung der allgemeinen Formel

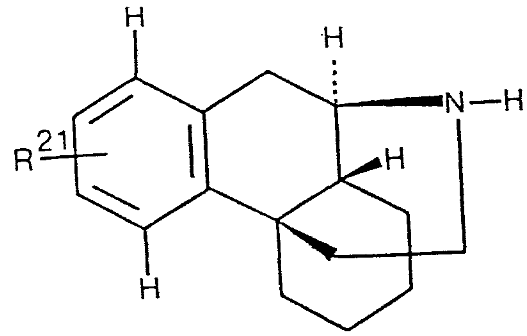

Ib

worin $R^{21}$ obige Bedeutung besitzt, mit einem eine niedere Alkyl-, niedere Alkenyl-, niedere Cycloalkyl-niedere Alkyl-, Cyano-niedere Alkyl-, Arylniedere Alkyl- oder Heteroaryl-niedere Alkylgruppe liefernden Alkylierungsmittel alkyliert, oder

d) die cyclische Ketalschutzgruppe in einer Verbindung der allgemeinen Formel

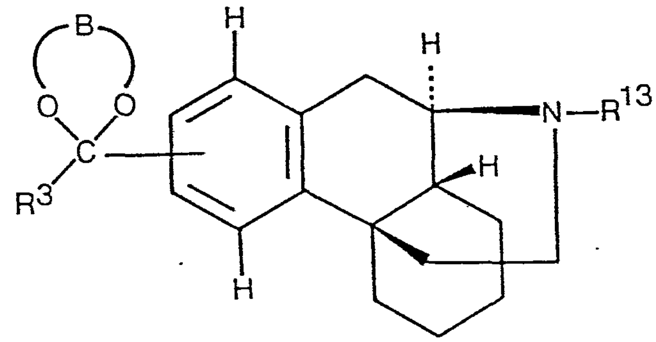

III

worin $R^{13}$ niederes Alkyl, niederes Cycloalkyl-niederes Alkyl, Aryl-niederes Alkyl oder Heteroaryl-niederes Alkyl, B Dimethylen oder Trimethylen und $R^3$ $(C_{1-6})$-Alkyl, Aryl oder Trifluormethyl bedeuten,

abspaltet, oder

e) die Carbonylgruppe in einer Verbindung der allgemeinen Formel

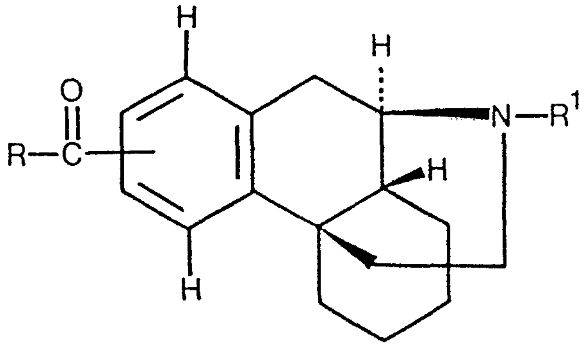

Ic

worin R und $R^1$ obige Bedeutung besitzen, reduziert, oder

f) die Carbonylgruppe in einer Verbindung der obigen Formel Ic alkyliert und

g) erwünschtenfalls eine erhaltene Verbindung der Formel I in ein pharmazeutisch annehmbares Säureadditionssalz überführt.

25. Arzneimittel enthaltend eine Verbindung gemäss einem der Ansprüche 1-17.

26. Analgetika, enthaltend eine Verbindung gemäss einem der Ansprüche 1-17.

***

Patentansprüche
OESTERREICH.

1. Verfahren zur Herstellung von Morphinanderivaten der allgemeinen Formel

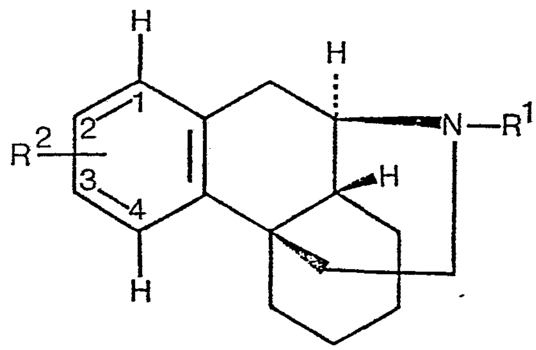

I

worin $R^1$ Wasserstoff, niederes Alkyl, niederes Alkenyl, niederes Cycloalkyl-niederes Alkyl, Cyano-niederes Alkyl, Aryl-niederes Alkyl oder Heteroaryl-niederes Alkyl, $R^2$ $(C_{2-7})$-Alkanoyl, Arylcarbonyl, Trifluormethylcarbonyl oder die Gruppe RR'C(OH)-, R $(C_{1-6})$-Alkyl und R' Wasserstoff oder niederes Alkyl bedeuten,

und von pharmazeutisch annehmbaren Säureadditionssalzen davon, dadurch gekennzeichnet, dass man

a) eine Verbindung der allgemeinen Formel

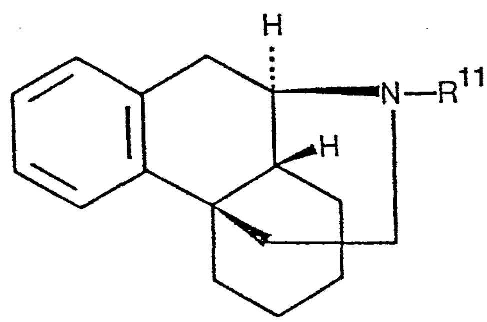

II

worin $R^{11}$ niederes Alkyl bedeutet,

acyliert, oder

b) eine Verbindung der allgemeinen Formel

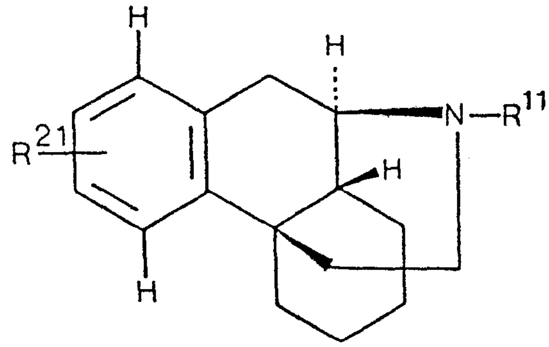

Ia

worin $R^{21}$ $(C_{2-7})$-Alkanoyl, Arylcarbonyl oder Trifluormethylcarbonyl bedeutet und $R^{11}$ obige Bedeutung besitzt,

N-dealkyliert, oder

c) eine Verbindung der allgemeinen Formel

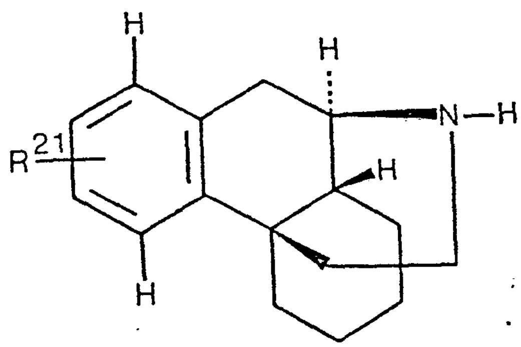

Ib

worin $R^{21}$ obige Bedeutung besitzt,

mit einem eine niedere Alkyl-, niedere Alkenyl-, niedere Cycloalkyl-niedere Alkyl-, Cyano-niedere Alkyl-, Arylniedere Alkyl- oder Heteroaryl-niedere Alkylgruppe liefernden Alkylierungsmittel alkyliert, oder

d) die cyclische Ketalschutzgruppe in einer Verbindung der allgemeinen Formel

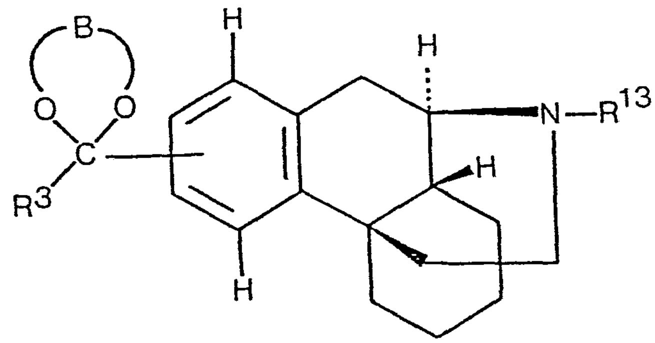

III

worin $R^{13}$ niederes Alkyl, niederes Cycloalkyl-niederes Alkyl, Aryl-niederes Alkyl oder Heteroaryl-niederes Alkyl, B Dimethylen oder Trimethylen und $R^3$ $(C_{1-6})$-Alkyl, Aryl oder Trifluormethyl bedeuten,

abspaltet, oder

e) die Carbonylgruppe in einer Verbindung der allgemeinen Formel

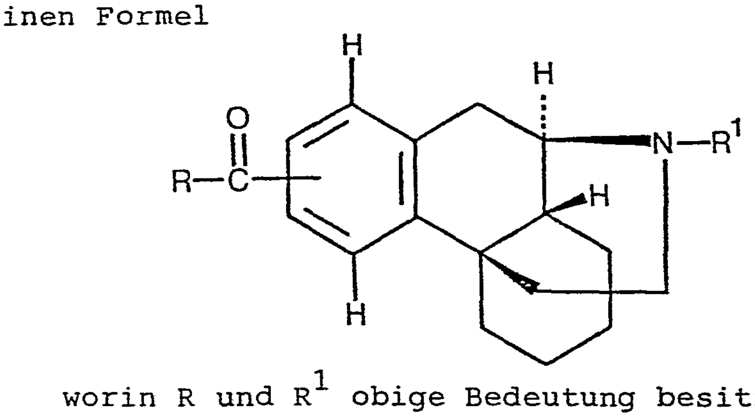

Ic

worin R und $R^1$ obige Bedeutung besitzen,

reduziert, oder

f) die Carbonylgruppe in einer Verbindung der obigen Formel Ic alkyliert und

g) erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass $R^2$ sich in Stellung 3 befindet.

3. Verfahren gemäss Anspruch 2, dadurch gekennzeichnet, dass $R^2$ ($C_{2-7}$)-Alkanoyl bedeutet.

4. Verfahren gemäss Anspruch 2 oder 3, dadurch gekennzeichnet, dass $R^1$ niederes Alkyl, niederes Alkenyl, niederes Cycloalkyl-niederes Alkyl oder Aryl-niederes Alkyl bedeutet.

5. Verfahren gemäss Anspruch 2, dadurch gekennzeichnet, dass $R^2$ die Gruppe RR'C(OH)-, R ($C_{1-6}$)-Alkyl und R' Wasserstoff oder niederes Alkyl bedeuten.

6. Verfahren gemäss Anspruch 5, dadurch gekennzeichnet, dass $R^1$ niederes Alkyl bedeutet.

7. Verfahren gemäss Anspruch 1, dadurch gekennzeichnet, dass man (-)-3-Acetyl-N-(cyclopropylmethyl)morphinan herstellt.

8. Verfahren gemäss Anspruch 1, dadurch gekennzeichnet, dass man (-)-3-(1-Hydroxyäthyl)-N-methylmorphinan herstellt.

9. Verfahren gemäss Anspruch 1, dadurch gekennzeichnet, dass man (-)-3-Acetyl-N-(cyclobutylmethyl)morphinan herstellt.

10. Verfahren gemäss Anspruch 1, dadurch gekennzeichnet, dass man (-)-3-Acetyl-N-methylmorphinan herstellt.

11. Verfahren gemäss Anspruch 1, dadurch gekennzeichnet, dass man (-)-3-Propionyl-N-methylmorphinan herstellt.

12. Verfahren gemäss Anspruch 1, dadurch gekennzeichnet, dass man (-)-3-(2-Hydroxy-2-propyl)-N-methylmorphinan herstellt.

13. Verfahren gemäss Anspruch 1, dadurch gekennzeichnet, dass man (-)-3-Acetyl-N-(3-methyl-2-butenyl)morphinan herstellt.

14. Verfahren gemäss Anspruch 1, dadurch gekennzeichnet, dass man (-)-3-Benzoyl-N-methylmorphinan herstellt.

15. Verfahren gemäss Anspruch 1, dadurch gekennzeichnet, dass man (-)-3-Acetyl-N-(2-phenäthyl)morphinan herstellt.

16. Verfahren gemäss Anspruch 1, dadurch gekennzeichnet, dass man (-)-3-Acetyl-N-(2-cyanoäthyl)morphinan herstellt.

17. Verfahren gemäss Anspruch 1, dadurch gekennzeichnet, dass man (-)-2-Acetyl-N-methylmorphinan herstellt.